(19) Europäisches Patentamt
European Patent Office
Office européen des brevets

(11) EP 2 628 713 A1

(12) **EUROPEAN PATENT APPLICATION**

(43) Date of publication:
**21.08.2013 Bulletin 2013/34**

(51) Int Cl.:
***C02F 3/34*** *(2006.01)* *C02F 101/34* *(2006.01)*

(21) Application number: **12155557.7**

(22) Date of filing: **15.02.2012**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicants:
- **Karl-Franzens-Universität Graz**
  **8010 Graz (AT)**
- **Technische Universität Graz**
  **8010 Graz (AT)**
- **ACIB GmbH**
  **8010 Graz (AT)**

(72) Inventors:
- **Kulterer, Martin**
  **8010 Graz (AT)**
- **Reichel, Victoria**
  **8010 Graz (AT)**
- **Ribitsch, Volker**
  **8010 Graz (AT)**
- **Glieder, Anton**
  **8200 Gleisdorf (AT)**
- **Krainer, Florian**
  **8010 Graz (AT)**

(74) Representative: **Gassner, Birgitta et al**
**REDL Life Science**
**Patent Attorneys**
**Donau-City-Straße 1**
**1220 Wien (AT)**

# (54) Degradation of hormones using recombinant HRP isoenzymes

(57) The present invention relates to the use of recombinant heme-containing horseradish peroxidase isoenzyme with improved technological properties such as altered glycosylation, improved catalytic properties or improved stability and different ranges of pH optima and improved surface interactions in the treatment of waste water.

EP 2 628 713 A1

**Description**

FIELD OF THE INVENTION

The present invention relates to the use of recombinant heme-containing horseradish peroxidase isoenzymes with improved properties in the treatment of waste water.

BACKGROUND OF THE INVENTION

Horseradish (*Armoracia rusticana*) is a herb cultivated worldwide in temperate regions, mainly due to the culinary use of its roots. However, the roots of *A. rusticana* are also used for the acquisition of highly versatile enzymes, the horseradish peroxidases (HRP) (Veitch, N.C. (2004) Phytochemistry 65, 249-259).

Peroxidases use hydrogen peroxide or various organic hydroperoxides as electron acceptors and are subsequently able to catalyze several oxidative reactions. They are encoded by various multigenic families, which are classified not only according to specific catalytic characteristics, but also according to structural properties and sequence information. A major mean for peroxidase taxonomy is the discrimination between heme-containing peroxidases and non-heme peroxidases. The latter contain five superfamilies, whereas the heme-containing peroxidases contain six superfamilies (Passardi, F., et al., (2007), Phytochemistry 68, 1605-11; Hofrichter, M., et al., (2010), Applied microbiology and biotechnology 87, 871-97).

All heme-containing peroxidases share some common features:

- A ferriprotoporphyrin IX prosthetic group at the active site with the key catalytic residues;
- Essential structural elements, such as two buried calcium-binding sites, four cysteine bridges and a buried salt-bridge;
- Hydrogen bonds and structural water molecules from the heme pocket to the distal calcium-binding site.

The non-animal heme peroxidases (synonymously referred to as plant peroxidases) comprise the majority of the so far identified heme peroxidase sequences. Several HRP isoenzymes have been described in literature. The horseradish peroxidase isoenzymes are referred to codes depending on their calculated isoelectric point (e.g. HRP A-isoenzymes have acidic isoelectric points, B- and C-isoenzymes are neutral or neutral-basic, D- and E-isoenzymes are basic).

The isoenzyme HRP C as mentioned in literature corresponds to the Peroxidase C1A chain which is part of the peptide encoded by the gene prxC1a (GenBank: M37156.1; (Fujiyama K. et. al (1988), European journal of biochemistry / FEBS 173, 681-7.)). The corresponding C1A UniProt entry annotates a N-terminal hydrophobic leader peptide (positions 1-30), the Peroxidase C1A chain (31-338) and a C-terminal propeptide part (339-353) that is believed to convey vacuolar targeting (UniProtKB: P00433) (Veitch, N.C. (2004) Phytochemistry 65, 249-259). Further HRP nucleotide sequences published in the GenBank database encoded the following known HRP isoenzymes: C1 C (M60729.1), C1 B (M37157.1), C3 (D90116.1), C2 (D90115.1) and N (X57564.1) (Kobayashi, Ket al. (1988) European journal of biochemistry / FEBS 173, 681-7, Fujiyama, K. et al. (1990) Gene 89, 163-9, Bartonek-Roxå, E et al. (1991) Biochimica et biophysica acta 1088, 245-50.*)*. These isoenzymes plus HRP A2 and E5 were also published at UniProt. The genes of nowadays published genomic DNA sequences encoding HRP isoenzymes are structured into four exons and three introns with identical splice site positions (Veitch, N.C. (2004) Phytochemistry 65, 249-259). Table 1 summarizes all isoenzymes published at UniProt or GenBank (effective March 2011):

Table 1

| isoenzyme | GenBank | UniProt | calculated IEP | calculated MW kDa |
|---|---|---|---|---|
| C1A | M37156.1 | P00433 | 5.67 | 38.82509 |
| C1B | M37157.1 | P15232 | 5.74 | 38.64586 |
| C1C | M60729.1 | P15233 | 6.21 | 36.54824 |
| C2 | D90115.1 | P17179 | 8.70 | 38.03538 |
| C3 | D90116.1 | P17180 | 7.50 | 38.17950 |
| A2 | - | P80679 | 4.72 | 31.89938 |
| E5 | - | P59121 | 9.13 | 33.72242 |
| N | X57564.1 | Q42517 | 7.48 | 35.12629 |

Applications of horseradish peroxidases are numerous and diverse. HRP is used in bioscience and biotechnology as well as in studies for medical applications.

A very common application of HRP is nowadays in biosensors, predominantly for the real-time quantification of $H_2O_2$ but also for the in vivo detection of glucose, ethanol or tumor markers via co-immobilization with a $H_2O_2$-producing enzyme.

Another application for horseradish peroxidases is in bioremediation, such as the detoxification of phenolic wastewater. Efforts are made to immobilize HRP in order to facilitate reusability and increase enzyme stability: HRP was used for the removal of natural and synthetic estrogens from wastewater.

The use of HRP enzymes for natural and synthetic estrogens-estrone (E1), 17β-estradiol (E2), estriol (E3), and 17α-ethinylestradiol (EE2)-removal has been described by Auriol et al. (Water Research 40 (2006) 2847-2856; Chemosphere 68 (2007) 1830-1837 and Chemosphere 70 (2008) 445-452). Specifically, once activated by the co-factor $H_2O_2$, HRP enzymes catalyze the oxidation of aqueous aromatic compounds - such as estrogens.

In order to reduce the operating costs associated with HRP enzyme treatment diverse efforts have been undertaken such as determination of HRP's affinity to different substrates, defining the optimum HRP and $H_2O_2$ doses or evaluation of the impact of the waste water matrix (Auriol 2007).

Thus, there is still a need for HRP isoenzymes with improved properties to reduce the costs associated with the HRP treatment.

## SUMMARY OF THE INVENTION

The present invention relates to the use of recombinant heme-containing horseradish peroxidase isoenzyme with improved technological properties such as altered glycosylation, improved catalytic properties or improved stability and different ranges of pH optima and improved surface interactions in the treatment of waste water.

The expression of recombinant plant peroxidases has been a matter of investigation since the early 1990s with the main focus lying on *E. coli* as expression host. However, the extremely low yields and formation of inclusion bodies turned out to be a major issue in the expression of eukaryotic proteins in *E. coli* and a lot of effort has been put into the dealing with this issue.

Alternatively to *E. coli,* various eukaryotic organisms have been used as expression hosts for plant peroxidases. Recombinant HRP has been expressed in an insect tissue culture via a baculovirus transfer vector as well as in *Saccharomyces cerevisiae.* In 2000, Morawski et al. (Protein engineering 13, 377-84) published the expression of HRP variants in *S. cerevisiae* and *Pichia pastoris* with significantly increased HRP activity in the culture supernatant. However the yields were still in the range of a few mg/L yeast culture. Further hosts for HRP expression are *Nicotiana tabacum, Spodoperta frugiperda* and *Beta vulgaris.*

Recombinantly expressed HRP has been subject to directed evolution in order to improve its expression and enzymatic properties such as specific activity or thermal stability. Morawski et al. (2000, 2001) described a 40-fold increase in specific activity after three rounds of random point mutagenesis and screening in a *S. cerevisiae* culture supernatant. The mutations were mainly located in either loop or surface regions. Even though the specific activity could be improved, the thermal stability was decreased. However, an additional single site mutation (N175S) led to a significant improvement in thermal stability.

Expression systems for expression of exogenous foreign genes in eukaryotic and prokaryotic cells are basic components of recombinant technology. Despite the abundance of expression systems and their wide-spread use, they all have characteristic disadvantages. For example, while expression in *E. coli* is probably the most popular as it is easy to grow and is well understood; eukaryotic proteins expressed therein are not properly modified. Moreover, those proteins tend to precipitate into insoluble aggregates and are difficult to obtain in large amounts.

Novel recombinant HRP isoenzymes with improved properties and method for producing them are disclosed in EP11185833.8.

Thus, the present invention encompasses the use of recombinant HRP iso-enzmes (or a functional derivatives thereof) in treatment of wastewater, specifically in the removal of endocrine disrupting compounds such as estrogens. In particular, the novel recombinant HRP isoenzymes show improved properties in the removal of natural and/or synthetic estrogens.

## DETAILED DESCRIPTION OF THE INVENTION

In one embodiment, the present invention relates to a process for the treatment of wastewater comprising the steps of:

a. adding at least one recombinant HRP isoenzyme to the wastewater;
b. allowing said at least one recombinant HRP isoenzyme to react with the wastewater for a predetermined duration

of time.

In one embodiment, the present invention relates to a process for the treatment of wastewater comprising the steps of:

a. adding at least one recombinant HRP isoenzyme to the wastewater;
b. allowing said at least one recombinant HRP isoenzyme to react with the wastewater for a predetermined duration of time, and optionally
c. quantifying the reaction to determine the extent of purification of the wastewater by means of the reduced level of the pollutants, and

wherein in said process is operated without $H_2O_2$.
In an aspect of this invention a process for the treatment of wastewater is provided, wherein said pollutants are selected from the group consting of endocrine-disrupting compounds, phenols, chlorinated phenols, cresols, aromatic amines. Important examples: degradation of TNT, PCBs, azo dyes, and fluorescent dyes.
In a further aspect of this invention a process for the treatment of wastewater is provided, wherein said endocrine-disrupting compounds are natural or synthetic estrogens.
In a further aspect of this invention a process for the treatment of wastewater is provided, wherein said pollutants are effectively removed.
In a further aspect of this invention a composition for the treatment of wastewater comprising at least one recombinant HRP isoenzymes or a mixture thereof, wherein said composition is devoid of the $H_2O_2$ cofactor is provided.
In a further aspect of this invention a composition for the treatment of wastewater is provided, wherein said at least one recombinant HRP isoenzyme is essentially purified.
In a further aspect of this invention a composition for the treatment of wastewater is provided, wherein said recombinant HRP isoenzmye is selected from the group consisting of C1A and A2A.
In a further aspect of this invention a composition for the treatment of wastewater is provided, wherein said recombinant HRP isoenzyme is A2A.
The invention also provides a solid support comprising at least one recombinant HRP isoenzyme immobilized thereon. In one embodiment, at least one recombinant HRP isoenzyme selected in the group consisting C1A and A2A as defined above, is immobilized on said solid support. In one aspect of this embodiment, the solid support of the invention further comprises at least one further immobilized enzyme.
Several techniques can be used to immobilize enzymes on solid supports, and are well-known by those skilled in the art. They are mainly based on chemical and physical mechanisms.
Said solid supports can include adsorbent materials such as activated carbon, resins or porous materials like macro-porous polymer materials. In a particular embodyiment of the invention, said solid support is CPC-silica beads or porous glass beads.
In another embodiment, said solid support is a membrane to which the enzyme is immobilized by covalent linkage. For example, said membrane can be a microfiltration membrane.
The solid support according to the invention can be used in a wide range of different settings. For example, the solid support can be packed in a column. Such a column can be installed within wastewater treatment plants to decontaminate wastewater and to remove endocrine disrupting compounds before they reach the environment. In another setting, the solid support can be used in packed-bed reactors with environmental or waste waters to be treated flowing through the reactor and being in contact with immobilized recombinant HRP isoenzymes.

DEFINITIONS

The term "effectively removed" refers to the effective removal of endocrine disrupting compound as determined by solid phase extraction (SPE)/fluorescence spectroscopy. SPE was performed using TC 18 reversed phase cartridges (Sigma Aldrich Steinheim, Germany). The amount of endocrine disrupting compounds in the treated waste water is below the detection limit (~50 μg/L) as determined by the assay, showing that more than 95% of EE2 was removed.
The term "essentially purified" refers to a purified recombinant isoenzyme that has been separated or isolated from the environment in which it was prepared. The term "essentially purified", with regard to a preparation of one or more recombinant HRP isoenzyme, refer to a preparation containing fewer than about 20%, more preferably fewer than about 15%, 10%, 8%, 7%, most preferably fewer than about 5%, 4%, 3%, 2%, 1%, or less than 1%, impurities.
The term "effective amount" denotes an amount suitable for disrupting endocrine compounds in the environment setting or waste material to be treated. These amounts can vary to a large extend depending on the concentration of the endocrine compounds, the activity of the recombinant HRP isoenzyme, the temperature, etc. The person skilled in the art is able to adapt the amount of recombinant HRP isoenzyme after having measured these parameters.

The term "pollutants" refers but is not limited to dyes, aromatic compounds, phenolics, nitrobenezenes, such as for examples degradation productds of TNT, PCBs, azo dyes, fluorescent dyes and endocrine disrupting compounds which encompass a variety of chemical classes, including drugs, pesticides, compounds used in the plastics industry and in consumer products, industrial by-products and pollutants, and even some naturally produced botanical chemicals.

DESCRIPTION OF THE DRAWINGS

Figure 1: Fluorescence emission spectra (Excitation: 280 nm) of EE2 in different solvents.
Figure 2: Fluorescence emission spectra (Excitation: 280 nm) of EE2 after incubation with different HRPs and SPE. The activity of HRP A2A was proven by testing the ultra pure HRP A2A enzyme.

The subject matter of the following definitions is considered embodiments of the present invention:

1. A process for the removal of pollutants in wastewater comprising the steps of:

a. adding at least one recombinant HRP isoenzyme to the wastewater;
b. allowing said at least one recombinant HRP isoenzyme to react with the wastewater for a predetermined duration of time; and optionally
c. quantifying the reaction to determine the extent of purification of the wastewater by means of the reduced the level of the pollutants, and

wherein in said process is operated without $H_2O_2$.
2. A process according to claim 1, wherein said pollutants are selected from the group consting of endocrine-disrupting compounds, phenols, chlorinated phenols, cresols, and aromatic amines.
3. The process acorrding to claim 2, wherein said endocrine-disrupting compounds are natural or synthetic estrogens.
4. The process according any one of claims 1 to 3, wherein said pollutants are effectively removed.
5. The process according to any one of claims 1 to 4, wherein said recombinant HRP isoenzyme is immobilized.
6. A composition for the treatment of wastewater comprising at least one recombinant HRP isoenzymes or a mixture thereof, wherein said composition is devoid of the $H_2O_2$ cofactor.
7. The composition according to claim 6, wherein said at least one recombinant HRP isoenzyme is essentially purified.
8. The composition according to claim 6 or 7, wherein said recombinant HRP isoenzmye is selected from the group of recombinant HRP isoenzymes, preferably from C1A and A2A.
9. The composition according to claim 8, wherein said recombinant HRP isoenzyme is A2A.
10. A solid support comprising immobilized thereon:

- at least one HRP recombinant isoenzyme, alone or in combination with
- one or more other enzymes.

11. The solid support according to claim 10, wherein said at least one recombinant HRP isoenzyme is selected in the group consisting of C1A and A2A

**Examples**

**Example 1. Production of HRP A2A in *Pichia pastoris***

**1.1. Perparation of media and buffer solution**

Production

500 x B: 10 mg / 50 mL d-Biotin, filter sterilized 10 x YNB: 134 g/L Difco™ yeast nitrogen base w/o amino acids, autoclaved. 10 x PPB: 30.0 g/L $K_2HPO_4$, 118.0 g/L $KH_2PO_4$. If needed, pH 6.0 is adjusted with 85% (v/v) o-phosphoric acid and 1 M KOH, autoclaved. 10 x GY: 100 mL/L ≥ 98% glycerol, 900 mL/L $dH_2O$, autoclaved ACSS (ammonium chloride stock solution): 250 g/L $NH_4Cl$, autoclaved PTM1 trace salts: 5 mL/L 95-98% $H_2SO_4$, 0.02 g/L $H_3BO_3$, 5.99 g/L $CuSO_4.5H_2O$, 0.92 g/L $CoCl_2.6H_2O$, 65.0 g/L $FeSO_4.7H_2O$, 3.0 g/L $MnSO_4.H_2O$, 1.18 g/L KJ, 0.2 g/L $Na_2MoO_4.2H_2O$, 42.18 g/L $ZnSO_4.7H_2O$, 995 mL/L $dH_2O$, filter sterilized BSM (basal salts medium): 40 g/L ≥ 98% glycerol, 0.17 g/L $CaSO_4.2H_2O$, 2.32 g/L $MgSO_4.7H_2O$, 2.86 g/L $K_2SO_4$, 4.25 mL/L ≥ 85 % o-phosphoric acid, 0.64 g/L KOH, 0.22 NaCl,

950 mL/L $dH_2O$. Addition of 4.35 mL/L PTM1 trace salts, 4.35 mL/L 500x B, 40 mL/L ACSS, 100 μL/L ACEPOL 83 E after autoclaving.

BMGY: 10 g/L Bacto™ yeast extract, 20 g/L Bacto™ peptone, 700 mL $dH_2O$, 100 mL 10 x YNB, 100 mL/L 10x PPB. Addition of 100 mL/L 10 x GY, 2 mL/L 500 x B after autoclaving.

Glycerol feed medium: 587.7 g ≥ 98% glycerol, add $dH_2O$ to 880 mL. Addition of 10.59 mL PTM trace salts, 10.59 mL 500 x B after autoclaving. Methanol feed medium: 1000 g ≥ 99.8% methanol. Addition of 15.17 mL 500 x B, 15.17 mL PTM1 trace salts after autoclaving.

<u>Purifcation (chromatography)</u>

Buffer HIC-A: 1 M $(NH_4)_2SO_4$, 20 mM Tris, pH 7.0 adjusted with 37% HCl Buffer HIC-B: 20 mM Tris, pH 7.0 adjusted with 37% HCl Buffer QFF-A: 50 mM Tris, pH 9.5 adjusted with 37% HCl Buffer QFF-B: 1 M NaCl, 50 mM Tris, pH 9.5 adjusted with 37 % HCl Buffer Superdex: 150 mM NaCl, 3.5 mM citrate, 32.9 mM $Na_2HPO_4$, pH 7.0

### 1.2. Production of HRP A2A in P. pastoris in a 5 L-BIOSTAT® bioreactor

A P. pastoris strain expressing HRP isoenzyme A2A was used for large-scale cultivation in a 5 L-BIOSTAT® bioreactor (Sartorius Stedim Biotech GmbH, Göttingen, Germany). Pre-cultures were grown in BMGY. The cultivation in a 5 L-BIOSTAT® bioreactor was performed in BSM, pH 5.0 was maintained by automatic addition of 25% ammonia solution.

With a starting OD600 ~ 1.0 in a 5 L-BIOSTAT® bioreactor, a Batch cultivation was run over night without any additional flow of glycerol feed medium. In a subsequent FedBatch cultivation, the flow of glycerol feed medium was increased to 30% of the max. pump capacity over 5 h, then linearly decreased to 0% over 3 h. In a subsequent inducing cultivation phase, the flow of methanol feed medium was linearly increased to 7.5% over 3 h, and then run for 60 h at this flow.

### 1.3. Purification of HRP A2A

<u>FPLC preparations</u>

FPLC runs were performed using an AKTA purifier (former Amersham Pharmacia Biotech, nowadays GE Healthcare Europe GmbH, Vienna, Austria). The Sartorius Vivaflow 50 system (30,000 MWCO cut-off) was used for sample concentration and buffer change. In order to concentrate samples of volumes smaller than 50 mL, the Sartorius Vivaspin 20 system (3,000 MWCO cut-off) was used. Prior to any FPLC run, all used solutions were filtered through cellulose acetate filters (0.2 μm pore size/Sartorius Mechatronics Austria GmbH, Vienna, Austria) using a diaphragm pump (VACUUBRAND GMBH + CO KG, Wertheim, Germany).

<u>Hydrophobic interaction chromatography</u>

For hydrophobic interaction chromatography, the buffer HIC-A was used as starting buffer. Buffer HIC-B was used for elution. Cleaning and equilibration of Phenyl Sepharose® 6 Fast Flow (high sub) (GE Healthcare Europe GmbH, Vienna, Austria) - packed in a XK26/20 column (former Amersham Pharmacia Biotech, nowadays GE Healthcare Europe GmbH, Vienna, Austria) - was done as recommended by the manufacturer. Elution was done by replacing buffer HIC-A with buffer HIC-B in a linear gradient from 0% HIC-B to 100% HIC-B over 23 x column volumes (CV) with a flowrate of 15 mL/min (~ 169.5 cm/h). Changes in absorption at 280 nm and 404 nm, conductivity and the concentration of buffer HIC-B on the column were followed with the UNICORN™ software. The collected fractions exposing HRP activity (with the ABTS assay) were pooled and used for the subsequent purification step.

<u>Anion exchange chromatography</u>

For anion exchange chromatography, the buffer QFF-A was used as starting buffer and buffer QFF-B was used for elution. 20 mL Q Sepharose® Fast Flow (GE Healthcare Europe GmbH, Vienna, Austria) were packed to a XK26/20 column, washed and equilibrated with buffer QFF-A in accordance with the manufacturer's recommendations. Elution was done by changing the ratio of QFF-A:QFF-B in a stepwise gradient. Changes in absorption at 280 nm and 404 nm, conductivity and the concentration of buffer QFF-B on the column were followed with the UNICORN™ software. The collected fractions exposing HRP activity (with the ABTS assay) were pooled and used for the final purification step.

Size exclusion chromatography

For size exclusion chromatography, the Superdex buffer was used. The column HiLoad™ 16/60 Superdex™ 200 prep grade 120 mL (GE Healthcare Europe GmbH, Vienna, Austria), prepacked with 120 mL matrix material, was washed and equilibrated as recommended by the manufacturer. The sample was concentrated using the Vivaspin 20 system and loaded onto the column with a flowrate of 0.3 mL/min (~ 9.0 cm/h). The same flowrate was applied for the size exclusion run over 2x CV. Changes in absorption at 280 nm and 404 nm and conductivity were followed with the UNICORN™ software. The fractions were checked for HRP activity with the ABTS assay, the fractions with highest HRP activities were used as purified solution of HRP isoenzyme A2A.

**1.4. Characterization of HRP A2A**

**1.4.1. Posttranslational modifications of HRP A2A**

In order to study the purified recombinant HRP A2A for posttranslational modifications, a mass spectrometric analysis at the ZMF Mass Spectrometry - Proteomics Core Facility was conducted. Further, the PredictProtein web tool was used on the mature HRP A2A amino acid sequence in order to compare the predicted results with those from the mass spectrometry.

Having a homogeneously glycosylated HRP A2A might significantly simplify eventual crystallization and structure analysis, which has been hindered so far by the N-glycans' heterogeneity of HRP A2 directly isolated from horseradish.

**1.4.2. Isoelectric focusing of HRP A2A**

The binding of HRP A2A to QFF at pH 9.5 happened at a much more basic pH as expected (considering the theoretical IEP of A2A at pH 4.82).

Thus, an isoelectric focusing of HRP A2A was performed and compared to the commercially available HRP preparations Type I, Type II, Type VI, Type VI-A, Type XII from Sigma-Aldrich Handels GmbH and a HRP preparation from Toyobo Co., Ltd. For isoelectric focusing, Novex® IEF gels were used. Protein samples were handled in accordance to the recommendations from Invitrogen™ life technologies. As a reference, 5 μL of the IEF Marker 3-10, Liquid Mix from SERVA Electrophoresis GmbH were used. Buffers and run conditions were used as recommended. After the run, the gel was fixed for 15 min in 12% TCA (50.0 g ≥ 99.5% trichloroacetic acid dissolved in 500 mL dH2O). For staining, the SimplyBlue™ SafeStain solution from Invitrogen™ life technologies was used as recommended. The isoelectric focusing of HRP A2A indicated an IEP at pH 3.5 - 4.2 which was close to the calculated theoretical IEP at pH 4.82. Most probably, the ionizable amino acid groups were predominantly not present on the enzyme's surface, which could explain its unexpected binding behavior towards QFF.

The IEP of the isoenzymes in the Sigma type I preparation was mainly around pH 5.0, the type II preparation showed two predominant isoenzymes at pH 5.3 - 6.0 and 3.5 - 4.2. The latter also seemed to be present in type VI, VI-A and XII with additional isoenzymes at 5.3 - 6.0 in type VI. The Toyobo preparation also showed an isoenzyme at pH 3.5 - 4.2, but slightly more basic than the A2A and the isoenzyme identified in the Sigma preparations.

**1.4.3. HRP A2A and ABTS**

pH optimum of HRP A2A plus ABTS

The interaction of HRP A2A was verified with the HRP standard substrate ABTS. The influence of pH on the catalysis of ABTS was studied. The ABTS assay was performed with the purified HRP A2A in buffers of different pH:

The 50 mM NaOAc, pH 4.5 standard buffer was used (Morawski et al.(2000)), 20 mM citrate-NaOH buffers at pH 3.0, 3.5, 4.0, 4.5, 5.0, 6.0, 7.0 and 20 mM Tris-HCl buffers at pH 7.0, 8.0, 9.0, 10.0. By performing the assay at the same pH with different buffer systems (i.e. 50 mM NaOAc and 20 mM citrate-NaOH at pH 4.5; 20 mM citrate-NaOH and 20 mM Tris-HCl at pH 7.0), any influences of the buffer systems on the reaction was excluded, other than the pH. Changing to the corresponding pH was achieved by 1:200 dilution of the purified A2A in the respective buffer.

The pH optimum for the conversion of ABTS by HRP A2A at pH 4.5 was successfully identified. Interestingly, Hiner et al. reported in 2001 a broad pH optimum of HRP A2 for the conversion of ABTS at pH 5.5 - 6.5 (Journal of biological inorganic chemistry: JBIC: a publication of the Society of Biological Inorganic Chemistry 6, 504-16). However, the HRP A2 used for that publication was the preparation HRP-5 from Biozyme Labs (Blaenavon, UK) and has just been identified as A2 by isoelectric focusing (but not by sequencing). Most likely, this HRP also differs from the present recombinant A2A in its glycosylation pattern, which might also influence its enzymatic properties. In addition, the verified

sequence used for the recombinant production of A2A differed from the published A2, which could also explain the varying results.

No activity could be detected at pH lower than 3.5 or higher than 9.0. Since HRP activity is also absent at pH lower than 3.5 or higher than 9.0 for the conversion of guaiacol, it might be that the enzyme itself is impaired in its activity at this pH.

Michaelis-Menten kinetics of HRP A2A plus ABTS

Since the optimal pH of the reaction of HRP A2A with ABTS was identified. A buffer at this pH was used for determining the kinetic constants Vmax and KM for the HRP catalyzed conversion of ABTS. Herefore the assay was performed with varying concentrations of ABTS: 0.1, 0.33, 0.5, 0.75, 1.0, 1.5, 2.0 and 3.0 mM. The measured activities in mAU/min were used to calculate the corresponding ABTS units/mL (1 ABTS unit is defined as the amount of enzyme that converts 1 μmol ABTS per minute) via the equation depicted below.

$$ABTS\ units/\mathrm{mL} = \frac{\frac{mAU}{min} * f}{\varepsilon * \mathrm{d}} * \frac{V}{v}$$

**Equation for the calculation of ABTS units per milliliter.**

f = dilution factor = 200.

$\varepsilon_{ABTS}$ = absorption coefficient of ABTS = 34,700 M-1 cm-1.

d = path length = 0.42 cm.

V = total reaction volume = 155 μL.

v = enzyme solution volume = 15 μL.

In order to be able to measure the increase in the absorption at 404 nm in the ABTS reaction the purified A2A 1:200 was diluted. The dilution factor f is taken into consideration in all calculations. The path length d was verified with the corresponding PathCheck function of the SoftMax Pro 4.8 software.

The specific activity of HRP A2A for ABTS was seeked to be verified. Hereto the purified A2A was quantified via the Pierce BCA Protein Assay Kit. Using this data the specific activity of HRP A2A in ABTS units/mg was calculated via dividing the ABTS units/mL by the protein concentration in μg/mL and multiplying with 1000.

The performed BCA assay for protein quantitation revealed that both, BSA and HRP-VI-A were suitable as standard proteins for calculating the concentration of the purified HRP A2A.

HRP A2A quantitation by using the BSA standard curve yielded a concentration of 56.9 ng/μL and 63.1 ng/μL by using the HRP VI-A standard curve, giving an average HRP A2A concentration of 60.0 ng/μL, which was used for all further calculations.

By plotting the calculated ABTS units/mg of the measured activities against the respective ABTS concentrations a saturation curve following Michaelis-Menten kinetics was obtained. Plotting of the reciprocal values of the reaction rates against the reciprocal values of the corresponding ABTS concentrations yielded the Lineweaver-Burk plot which allowed the determination of Vmax and KM with ABTS as substrate by calculating the reciprocal negative value of the crossing point of the trendline and the x-axis (i.e. KM) and the reciprocal value of the crossing point of the trendline and the y-axis (i.e. Vmax):

$$\mathrm{Vmax} = 793\ \mathrm{ABTS\ units/mg}$$

$$\mathrm{KM} = 0.44\ \mathrm{mM\ ABTS.}$$

As for the pH profile of HRP A2A, the values Hiner et al. published for their A2 isoenzyme differed from this one: They reported a Vmax = 1,432 ABTS units/mg and a KM = 4.0 mM at pH 4.5 (34). The HRP A2A was already in

saturation at a concentration of 3.0 mM ABTS. Interestingly, this means a two times faster maximum conversion speed, but a ten times lower substrate affinity of their isoenzyme.

**1.4.4. HRP A2A and guaiacol**

pH optimum of HRP A2A plus guaiacol

Similarly to the verification of the pH optimum of the HRP-catalyzed ABTS reaction, another assay was performed in buffers of different pH with a second standard substrate, guaiacol. As standard buffer 20 mM phosphate-KOH buffer, pH 7.0 as published by Morawski et al. (2000) was tested. Moreover, further 20 mM phosphate-KOH buffers were tested at pH 5.0, 6.0 and 8.0, 20 mM citrate-NaOH buffers at pH 3.0, 3.5, 4.0, 4.5, 5.0, 6.0, 7.0 and 20 mM Tris-HCl buffers at pH 7.0, 8.0, 9.0, 10.0 and 10.5.

The pH optimum for the conversion of guaiacol by HRP A2A at pH 5.0 was verified, however the measured activities stayed at comparable levels until pH 7.0. In contrast to the corresponding ABTS profile, the enzyme's pH optimum seemed to dominate over the assay's pH optimum, since the profile much more resembled a normal distribution. Similarly to the ABTS profile, no significant activity could be measured below pH 3.5 or above pH 9.0.

Contrary to the pH profile with ABTS, the findings for the guaiacol pH profile matched the data published by Hiner et al. (2001). They also reported similar results for the conversion of guaiacol with HRP C1A. Probably, the guaiacol pH profile generally does not show bigger variations among the isoenzymes, which could explain the differences between A2A and their A2 in the ABTS assay, but not in the guaiacol assay. As for the pH profile of ABTS, the dependence of the absorption spectrum of guaiacol on pH was neglected and remains to be verified in further studies.

Michaelis-Menten kinetics of HRP A2A plus guaiacol

Using the gathered data on the optimal pH for the conversion of guaiacol by HRP A2A kinetic studies were performed in analogy to those of A2A with ABTS with the following accommodations in the calculations:

$$f = \text{dilution factor} = 65 \text{ and}$$

$$\varepsilon_{guaiacol} = \text{absorption coefficient of guaiacol} = 26{,}000\ M^{-1}\ cm^{-1}.$$

Thus guaiacol units were aimed to be obtained corresponding to varying concentrations of the substrate (0.5, 0.75, 1.0, 3.0, 5.0, 7.0, 10.0, 20.0 mM). 1 guaiacol unit is defined as the amount of enzyme that converts 1 $\mu$mol guaiacol per minute.

As for the graphical representation and determination of the kinetic constants of A2A with ABTS, the calculated guaiacol units/mg of the measured activities were plotted against the corresponding substrate concentrations and thus obtained a curve that goes into saturation at a certain guaiacol concentration. Vmax and KM for the conversion of guaiacol by HRP A2A were again determined by creating a Lineweaver-Burk plot:

$$Vmax = 59 \text{ guaiacol units/mg},$$

$$KM = 1.03 \text{ mM guaiacol}.$$

Hiner et al. (2001) published a comparable Vmax = 81 guaiacol units/mg for the conversion of guaiacol by their HRP A2. Unlike for the conversion of ABTS, their isoenzyme seems to show catalytic properties towards guaiacol that are comparable to the obtained data with recombinant A2A.

**1.4.5. Stability studies with HRP A2A**

Optimal storage pH of HRP A2A

The influence of pH on the storage stability of HRP A2A was evaluated. Therefore, the purified HRP A2A was

diluted 1:20 in buffers of different pH (20 mM citrate-NaOH buffers at pH 3.0, 4.0, 5.0, 6.0 and 7.0 and 20 mM Tris-HCl buffers at pH 8.0, 9.0 and 10.0) and stored the different tubes at 4 °C. In order to measure the starting / remaining activities at the time points 0 d, 2 d, 6 d, 10 d, 20 d (for pH 3.0 also measured after 1 d), and at the same assay conditions, aliquots from the tubes were further diluted with the different pHs 1:10 in 50 mM NaOAc buffer, pH 4.5 and the ABTS assay performed.

The activity of HRP A2A seems to be best preserved in buffers of pH 7.0 - 10.0 (see table 2). In pH 3.0 and 4.0, the HRP activity was completely abrogated; in pH 5.0 and 6.0, the activity was significantly decreased; in pH 7.0 - 10.0, the activity was equivalently well preserved.

Table 2

| pH | residual HRP activity after 20 d % | pH | residual HRP activity after 20 d % |
|---|---|---|---|
| 3.0 | 0.0 | 7.0 | 76.7 |
| 4.0 | 0.0 | 8.0 | 77.7 |
| 5.0 | 10.4 | 9.0 | 80.6 |
| 6.0 | 50.9 | 10.0 | 77.3 |

AT pH 3.0, the HRP activity is completely abrogated in short time (no activity measurable after 1 d). At pH 4.0, the activity also continuously decreased and was below detection limits after 10 d. However, even at this relatively low pH still a third of its original activity was detected after two days. HRP A2A still yielded measurable, but significantly decreased activity after 20 d in pH 5.0, and with a further descending trend. In pH 6.0, the activity was decreased to 50% of the starting activity after 20 d, however it seemed to stay stable at this level. The best preserved HRP activity was detected in buffers of pH 7.0 - 10.0. The residual HRP activity in these four buffers was approximately 78% of the initial activity and stayed stable at this level.

<u>Temperature stability of HRP A2A</u>

Pure A2A 1:200 (i.e. 0.3 ng/μL) was diluted in 50 mM NaOAc buffer, pH 4.5 and incubated at different temperatures: 20 °C, 37 °C, 50 °C, 65 °C. For each incubation temperature aliquots were taken after several time points and stored on ice until the end of the whole experiment. Finally, the HRP activities from all time points of one incubation temperature were measured at once with the ABTS assay.

The 0.3 ng/μL diluted HRP A2A met the expectations and lost its activity over time the faster, the higher the incubation temperature was. Storage for 60 min at 4 °C decreased the activity to 75% of the starting activity (4 °C data was taken from the experiment investigating the influence of protein concentration on stability, see below; data from 0.38 ng/μL HRP A2A). After 60 min, the diluted HRP A2A showed 50% of the initial activity with a descending trend at room temperature (i.e. 20 °C). At 37 °C, HRP activity was measurable for 30 min. At 50 °C and 65 °C, the activity was abrogated after 10 min and 3 min, respectively.

The finding, that HRP activity decreased relatively fast even at 4 °C did not match the observations on HRP stability made so far: Not any significant loss of activity was seen over months of HRP A2A stored at 4 °C. However, contrary to the A2A used for this temperature stability study, the stored A2A was undiluted (i.e. 60 ng/μL), hence it is hypothesized, that protein concentration might play a crucial role in HRP stability (see below).

<u>Dependence of the stability of HRP A2A on protein concentration</u>

HRP A2A stays stable for long time (>> 20 d) as long as it is undiluted. The purified HRP A2A was diluted with buffer (3.5 mM citrate, 32,9 mM Na2HP04, pH 7.0) to the following concentrations: 60.0 ng/μL, 2.0 ng/μL, 1.2 ng/μL, 0.8 ng/μL, 0.6 ng/μL, 0.46 ng/μL and 0.38 ng/μL. The HRP activity was measured after 0 h, 1 h, 5 h, 1 d, 3 d and 10 d. Hereto, aliquots were taken from the various dilutions and separately further diluted to a final total dilution of 1:200 (i.e. 0.3 ng/μL) and the ABTS assay performed.

A 6.7 ng/μL concentrated solution of HRP A2A was examined for the existence of nanoparticles using the ZetaPlus Zeta Potential Analyzer.

It is assumed that the stability of HRP A2A depends on the protein concentration of the solution it is dissolved in. By studying the HRP activities over time at different concentrations, the hypothesis was successfully confirmed and the crucial "point-of-no-return" in protein concentration identified above which HRP stability is guaranteed, and below which HRP activity decreases rather fast.

After storing HRP A2A at 4 °C for 10 d in concentrations ≥ 0.6 ng/μL no significant loss of HRP activity could

be detected. However the two lower concentrated HRPs completely lost activity within one day.

**Example 2: Removal of endocrine disrupting compounds**

**Methods/Procedures/Analytical tools**

*Preparation of 17 α Ethinylestradiol solution*

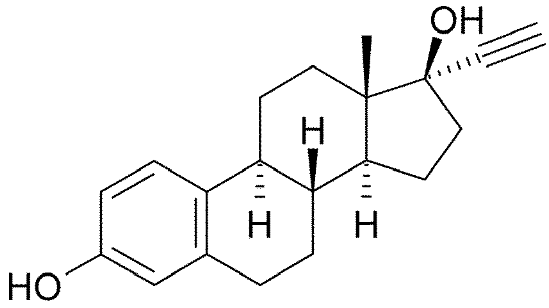

The saturation concentration of 17 α ethinylestradiol (EE2) in water is 11.3 mg/L. However, it was not possible to prepare aqueous solutions of satisfying concentration directly. Therefore, aqueous EE2 solution (5 mg/L) was prepared as follows: First, EE2 was dissolved in absolute ethanol (0.5 g/L). Next, 1 mL of this solution was added to 99 mL of bidistilled water under continous stirring.

**Enzyme and hormone incubation procedure**

150 μL fermentation bulk (average HRP content: 0.8 mg/L) solution or purified enzyme solution (HRP content: 34 mg/L) was added to 1.4 mL of an aqueous 10 mM $CaCl_2$ EE2 (5 mg/L) solution. Next, the mixture was incubated for 10 to 120 min under continuous shaking.

Following this procedure, the activity of two different isoenzymes against EE2, (C1A, and A2A) was tested.

**Determination of the enzyme activity**

Fluorescence spectroscopy measurements

In order to analyze the degradation process by spectroscopy, EE2 and its degradation products had to be separated from the enzymes. This was necessary because these substances exhibit very similar, overlapping UV absorption spectra as well as fluorescence emission spectra.

In order to find specific, intense signals, the emission spectra were recorded at excitation wavelengths ranging from 220 nm to 380 nm.

Moreover, the fluorescence emission spectra of pure EE2 (5 mg/L) and enzyme (0.8 mg/L), dissolved in bidistilled water as well as in aqueous 10 mM $CaCl_2$ solutions, were measured in order to investigate the influence of solutes on the intrinsic fluorescence (Figure 1).

**Results and Discussion**

Fluorescence spectroscopy

The amount of EE2 in solution was determined using fluorescence spectroscopy, because the technique provides high sensitivity, low background, and is very specific. The emission maximum of EE2 was found at 310 nm (280 nm excitation wavelength).

Unfortunately, the spectra of EE2, HRP, and impurities are very similar. It was not possible to find characteristic EE2 peaks which differed enough from the HRP spectra. Moreover, the solvent had a significant influence on the on the fluorescence spectra (Figure 1). The presence of $CaCl_2$ (co-factor) yielded a strong decrease of the maximum fluorescence intensity.

Therefore a way for separating EE2 from HRP and impurities had to find. Best results were obtained using solid phase extraction (SPE).

Solid phase extraction (SPE)

A reverse phase RP 18 material was used as stationary phase. After conditioning the cartridge, the sample was applied. The elution was performed in three steps. First, bidistilled water was used in order to remove hydrophilic components. Next, the column was rinsed with a solvent mixture of medium polarity in order to remove moderate hydrophilic components. Finally, EE2 was eluted with a more unpolar solvent mixture. Each step was optimized by varying the solvent mixtures and the elution volumes.

Enzyme activity test

After applying an analytical procedure basing on SPE and fluorescence spectroscopy, we tested two different recombinant HRP isoenzymes on their enzyme degradation efficiency. The presence of $H_2O_2$ is usually required for hormone degradation. It acts as electron acceptor in the catalytic cycle. However, the enzyme activity was tested in the absence of $H_2O_2$ because it was intended to avoid the addition of substances which are not present in waste or surface water. The second required co-factor are $Ca^{2+}$ ions, which is part of the active center. Therefore, the tests were performed in 10 mM $CaCl_2$ solutions. A buffer was not used, since the novel recombinant HRP isoenzymes are known to be active over a broad pH range (Figure 2).

The light grey curve is the spectra of pure EE2 after SPE. High activities (lowest intensities) were obtained for both tested recombinant HRP isoenzymes (C1A, A2A). Although C1A showed to be more active, HRP A2A was chosen. HRP C1A is highly glycosylated which makes the enzyme purification more difficult, time consuming and expensive whereas the purification of the low glycosylated HRP A2A is straight forward and avoids such negative implications.

**Conclusions**

After the set-up of a suitable analytical procedure it has been proven that recombinant HRP isoenzymes can be used for degrading EE2 very efficiently under environmental conditions.

**Claims**

**1.** A process for the removal of pollutants in wastewater comprising the steps of:

a. adding at least one recombinant HRP isoenzyme to the wastewater;
b. allowing said at least one recombinant HRP isoenzyme to react with the wastewater for a predetermined duration of time; and optionally
c. quantifying the reaction to determine the extent of purification of the wastewater by means of the reduced the level of the pollutants, and

wherein in said process is operated without $H_2O_2$.

**2.** A process according to claim 1, wherein said pollutants are selected from the group consting of endocrine-disrupting compounds, phenols, chlorinated phenols, cresols, and aromatic amines.

**3.** The process acorrding to claim 2, wherein said endocrine-disrupting compounds are natural or synthetic estrogens.

**4.** The process according any one of claims 1 to 3, wherein said pollutants are effectively removed.

**5.** The process according to any one of claims 1 to 4, wherein said recombinant HRP isoenzyme is immobilized.

**6.** A composition for the treatment of wastewater comprising at least one recombinant HRP isoenzymes or a mixture thereof, wherein said composition is devoid of the $H_2O_2$ cofactor.

**7.** The composition according to claim 6, wherein said at least one recombinant HRP isoenzyme is essentially purified.

**8.** The composition according to claim 6 or 7, wherein said recombinant HRP isoenzmye is selected from the group

of recombinant HRP isoenzymes, preferably from C1A and A2A.

**9.** The composition according to claim 8, wherein said recombinant HRP isoenzyme is A2A.

**10.** A solid support comprising immobilized thereon:

- at least one HRP recombinant isoenzyme, alone or in combination with
- one or more other enzymes.

**11.** The solid support according to claim 10, wherein said at least one recombinant HRP isoenzyme is selected in the group consisting of C1A and A2A

**Figure 1:**

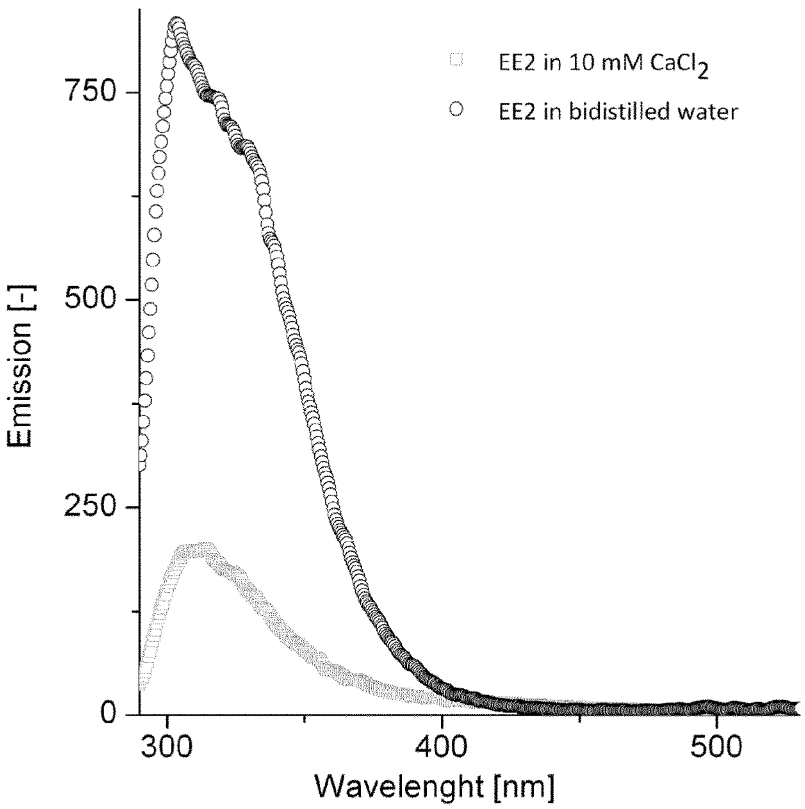


**Figure 2:**

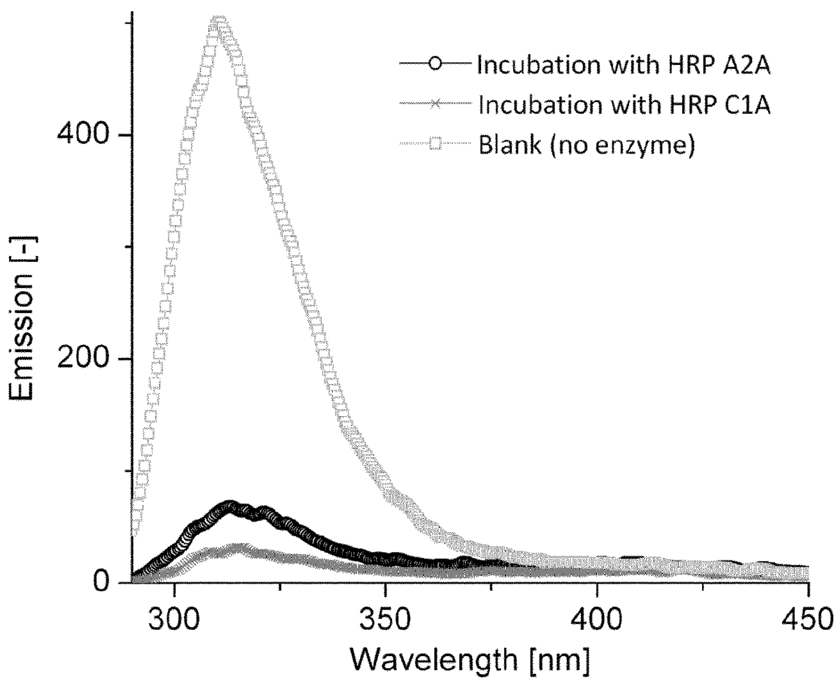

Europäisches Patentamt
European Patent Office
Office européen des brevets

# EUROPEAN SEARCH REPORT

Application Number
EP 12 15 5557

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ROJAS-MELGAREJO F ET AL: "Cinnamic carbohydrate esters show great versatility as supports for the immobilization of different enzymes", ENZYME AND MICROBIAL TECHNOLOGY, STONEHAM, MA, US, vol. 38, no. 6, 1 April 2006 (2006-04-01), pages 748-755, XP027948586, ISSN: 0141-0229 [retrieved on 2006-04-01] * abstract * * paragraph [3.3.1] * * paragraph [03.4] * * paragraph [0004] * ----- | 1-11 | INV. C02F3/34 ADD. C02F101/34 |
| X | MORAWSKI B ET AL: "Functional expression and stabilization of horseradish peroxidase by directed evolution in Saccharomyces cerevisiae", BIOTECHNOLOGY AND BIOENGINEERING, WILEY & SONS, HOBOKEN, NJ, US, vol. 76, no. 2, 1 September 2001 (2001-09-01), pages 99-107, XP002478995, ISSN: 0006-3592, DOI: 10.1002/BIT.1149 * page 107 * ----- | 1 | TECHNICAL FIELDS SEARCHED (IPC) C02F |
| X | RYAN ET AL: "Horseradish and soybean peroxidases: comparable tools for alternative niches?", TRENDS IN BIOTECHNOLOGY, ELSEVIER PUBLICATIONS, CAMBRIDGE, GB, vol. 24, no. 8, 1 August 2006 (2006-08-01), pages 355-363, XP005554426, ISSN: 0167-7799 * page 359, last paragraph * ----- | 1,2 | |
| | -/-- | | |

1

The present search report has been drawn up for all claims

| Place of search | Date of completion of the search | Examiner |
|---|---|---|
| The Hague | 24 August 2012 | González Arias, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 12 15 5557

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | AURIOL ET AL: "Natural and synthetic hormone removal using the horseradish peroxidase enzyme: Temperature and pH effects", WATER RESEARCH, ELSEVIER, AMSTERDAM, NL, vol. 40, no. 15, 1 August 2006 (2006-08-01), pages 2847-2856, XP005590001, ISSN: 0043-1354, DOI: 10.1016/J.WATRES.2006.05.032 * abstract * * paragraph [03.6] * ----- | 1-11 | |

TECHNICAL FIELDS SEARCHED (IPC)

The present search report has been drawn up for all claims

1

| Place of search | Date of completion of the search | Examiner |
|---|---|---|
| The Hague | 24 August 2012 | González Arias, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 11185833 A


### Non-patent literature cited in the description


- **VEITCH, N.C.** *Phytochemistry,* 2004, vol. 65, 249-259
- **PASSARDI, F. et al.** *Phytochemistry,* 2007, vol. 68, 1605-11
- **HOFRICHTER, M. et al.** *Applied microbiology and biotechnology,* 2010, vol. 87, 871-97
- **FUJIYAMA K.** *European journal of biochemistry / FEBS,* 1988, vol. 173, 681-7
- **KOBAYASHI, KET.** *European journal of biochemistry / FEBS,* 1988, vol. 173, 681-7
- **FUJIYAMA, K. et al.** *Gene,* 1990, vol. 89, 163-9
- **BARTONEK-ROXÅ, E et al.** *Biochimica et biophysica acta,* 1991, vol. 1088, 245-50
- **AURIOL et al.** *Water Research,* 2006, vol. 40, 2847-2856
- *Chemosphere,* 2007, vol. 68, 1830-1837
- *Chemosphere,* 2008, vol. 70, 445-452
- **MORAWSKI et al.** *Protein engineering,* 2000, vol. 13, 377-84